# FASCICULE DE BREVET EUROPEEN

(11) **EP 0 790 818 B1**
(45) Date de publication et mention de la délivrance du brevet: **30.05.2001**
(21) Numéro de dépôt: 96932626.3
(22) Date de dépôt: 20.09.1996
(51) Int. Cl.: A61K 7/13

(54) **COMPOSITION DE TEINTURE D'OXYDATION DES FIBRES KERATINIQUES ET PROCEDE DE TEINTURE METTANT EN OEUVRE CETTE COMPOSITION**
ZUSAMMENSETZUNGEN ZUM OXIDATIVEN FARBEN VON KERATINFASERN, UND VERFAHREN ZUM FARBEN UNTER VERWENDUNG DIESER ZUSAMMENSETZUNG
KERATIN FIBRE OXIDATION DYEING COMPOSITION AND DYEING METHOD USING SAME

(30) Priorité: 25.09.1995 FR 9511223
(43) Date de publication de la demande: 27.08.1997
(73) Titulaire: L'OREAL, 75008 Paris (FR)
(72) Inventeur: AUDOUSSET, Marie-Pascale, F-92600 Asnières (FR)
(74) Mandataire: Miszputen, Laurent
(86) Numéro de dépôt international: FR9601472
(87) Numéro de publication internationale: WO9711673

(56) Documents cités:
- EP-A- 0 428 441
- EP-A- 0 465 340
- WO-A-93/09759
- DE-A- 1 916 139
- DE-A- 3 031 709

## Description

La présente invention a pour objet une composition prête à l'emploi, pour la teinture d'oxydation des fibres kératiniques, en particulier des fibres kératiniques humaines telles que les cheveux, comprenant, dans un milieu acide (pH < 7), au moins une base d'oxydation, en association avec la 4-hydroxyindoline à titre de coupleur et un agent oxydant, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés indoliques ou indoliniques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin. Toutefois, les milieux alcalins présentent pour inconvénient d'entraîner une dégradation non négligeable des fibres. A titre d'exemple, il a déjà été proposé dans la demande de brevet français FR 2 008 797, des compositions pour la teinture d'oxydation des fibres kératiniques en milieu alcalin comprenant au moins une base d'oxydation et au moins un dérivé indolinique à titre de coupleur.

La coloration d'oxydation des fibres kératiniques peut également être réalisée en milieu acide, afin de limiter la dégradation des fibres kératiniques. Toutefois, les colorations obtenues dans ces conditions sont malheureusement généralement moins puissantes et moins tenaces que celles obtenues en milieu alcalin.

Or, la demanderesse vient maintenant de découvrir qu'il est possible d'obtenir de nouvelles teintures en milieu acide, capables d'engendrer des colorations puissantes, peu sélectives et résistant bien aux diverses agressions que peuvent subir les cheveux, en associant au moins une base d'oxydation, de la 4-hydroxyindoline et un agent oxydant et ceci de manière telle que le mélange résultant prêt à l'emploi présente un pH inférieur à 7.

Cette découverte est à la base de la présente invention.

L'invention a donc pour premier objet une composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation,
- de la 4-hydroxyindoline et/ou au moins l'un des ses sels d'addition avec un acide, à titre de coupleur,
- au moins un agent oxydant,
le pH de cette composition prête à l'emploi étant inférieur à 7.

Les colorations obtenues avec les compositions tinctoriales prêtes à l'emploi conformes à l'invention sont au moins aussi, sinon plus puissantes que celles de l'art antérieur obtenues avec les mêmes compositions mises en oeuvre à pH basique, et présentent par ailleurs une faible sélectivité et d'excellentes propriétés de résistances à la fois vis à vis des agents atmosphériques tels que la lumière et les intempéries et vis à vis de la transpiration et des différents traitements que peuvent subir les cheveux (lavages, déformations permanentes).

L'invention a également pour objet un procédé de teinture d'oxydation des fibres kératiniques mettant en oeuvre cette composition tinctoriale prête à l'emploi.

Les bases d'oxydation utilisables dans les compositions prêtes à l'emploi conformes à l'invention sont de préférence choisies parmi les paraphénylènediamines, les bis-phénylalkylènediamines, les para-aminophénols, les ortho-aminophénols, les bases hétérocycliques, et leurs sels d'addition avec un acide.

Les sels d'addition avec un acide utilisables dans le cadre des compositions tinctoriales de l'invention (bases d'oxydation et 4-hydroxyindoline) sont notamment choisis parmi les chlorhydrates, les bromhydrates, les sulfates et les tartrates.

Parmi les paraphénylènediamines utilisables à titre de bases d'oxydation dans le cadre des compositions prêtes à l'emploi conformes à l'invention, on peut notamment citer les composés répondant à la formule (I) suivante, et leurs sels d'addition avec un acide : 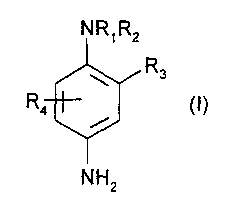 dans laquelle :
R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), phényle ou 4'-aminophényle,
R₂ représente un atome d'hydrogène, un radical alkyle en C₁-C₄, monohydroxyalkyle en C₁-C₄ ou polyhydroxyalkyle en C₂-C₄,
R₃ représente un atome d'hydrogène, un atome d'halogène tel qu'un atome de chlore, de brome, d'iode ou de fluor, un radical alkyle en C₁-C₄, monohydroxyalkyle en C₁-C₄ ou hydroxyalcoxy en C₁-C₄,
R₄ représente un atome d'hydrogène ou un radical alkyle en C₁-C₄.

Parmi les paraphénylènediamines de formule (I) ci-dessus, on peut plus particulièrement citer la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 4-amino N-(β-méthoxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, et leurs sels d'addition avec un acide.

Parmi les paraphénylènediamines de formule (I) ci-dessus, on préfère tout particulièrement la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 4-amino N-(β-méthoxyéthyl) aniline, la 2-chloro paraphénylènediamine, et leurs sels d'addition avec un acide.

Parmi les bis-phénylalkylènediamines utilisables à titre de bases d'oxydation dans le cadre des compositions prêtes à l'emploi conformes à l'invention, on peut notamment citer les composés répondant à la formule (II) suivante et leurs sels d'addition avec un acide : 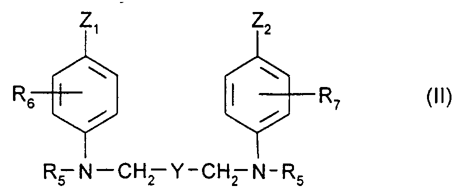 dans laquelle :
Z₁ et Z₂, identiques ou différents, représentent un radical hydroxyle ou NHR₈ dans lequel R₈ représente un atome d'hydrogène ou un radical alkyle en C₁-C₄,
R₅ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₆ et R₇, identiques ou différents, représentent un atome d'hydrogène ou d'halogène ou un radical alkyle en C₁-C₄,
Y représente un radical pris dans le groupe constitué par les radicaux suivants : 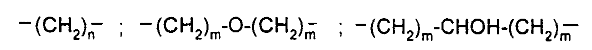 et 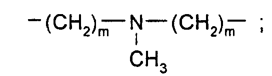 dans lesquels n est un nombre entier compris entre 0 et 8 inclusivement et m est un nombre entier compris entre 0 et 4 inclusivement.

Parmi les bis-phénylalkylènediamines de formules (II) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi ces bis-phénylalkylènediamines de formule (II), le N,N'-bis-(β-hydroxyéthyl) N,N'-bis-(4'-aminophényl) 1,3-diamino propanol ou l'un de ses sels d'addition avec un acide sont particulièrement préférés.

Parmi les para-aminophénols utilisables à titre de bases d'oxydation dans le cadre des compositions prêtes à l'emploi conformes à l'invention, on peut notamment citer les composés répondant à la formule (III) suivante et leurs sels d'addition avec un acide : 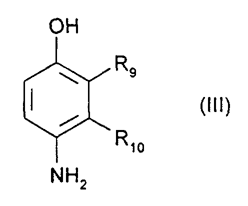 dans laquelle :
R₉ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₁₀ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄),
étant entendu qu'au moins un des radicaux R₉ ou R₁₀ représente un atome d'hydrogène.

Parmi les para-aminophénols de formule (III) ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

Parmi les orthoaminophénols utilisables à titre de bases d'oxydation dans le cadre des compositions prêtes à l'emploi conformes à l'invention, on peut plus particulièrement citer le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques utilisables à titre de bases d'oxydation dans le cadre des compositions prêtes à l'emploi conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diaminopyridine, et leurs sels d'addition avec un acide.
Parmi dérivés pyrimidiniques, on peut plus particulièrement citer les composés décrits par exemple dans les brevets allemand DE 2 359 399 ou japonais JP 88-169 571 et JP 91-333 495, comme la 2,4,5,6-tétra-aminopyrimidine, la 4-hydroxy 2,5,6-triaminopyrimidine, et leurs sels d'addition avec un acide.
Parmi les dérivés pyrazoliques, on peut plus particulièrement citer les composés décrits dans les brevets DE 3 843 892, DE 4 133 957 et demandes de brevet WO 94/08969 et WO 94/08970 comme le 4,5-diamino 1-méthyl pyrazole, le 3,4-diamino pyrazole, le 4,5-diamino 1-(4'-chlorobenzyl) pyrazole, et leurs sels d'addition avec un acide.

La ou les bases d'oxydation représentent de préférence de 0,0005 à 12 % en poids environ du poids total de la composition tinctoriale, et encore plus préférentiellement de 0,005 à 6 % en poids environ.

La 4-hydroxyindoline et/ou ses sels d'addition avec un acide représente(nt) de préférence de 0,0005 à 5 % en poids environ du poids total de la composition tinctoriale, et encore plus préférentiellement de 0,005 à 3 % en poids environ.

Selon une caractéristique essentielle de la présente invention, le pH de la composition tinctoriale prête à l'emploi conforme à l'invention est inférieur à 7 et peut varier de préférence entre 3 et 6,9. Il peut être ajusté à la valeur désirée au moyen d'agents acidifiants ou éventuellement alcalinisants habituellement utilisés en teinture des fibres kératiniques.

Parmi les agents acidifiants on peut citer, à titre d'exemple, les acides minéraux ou organiques comme l'acide chlorhydrique, l'acide orthophosphorique, les acides carboxyliques comme l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (IV) suivante : 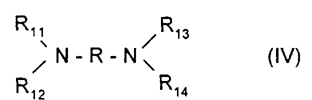 dans laquelle R est un reste propylène éventuellement substitué par un groupement hydroxyle ou un radical alkyle en C₁-C₄ ; R₁₁, R₁₂, R₁₃ et R₁₄, identiques ou différents, représentent un atome d'hydrogène, un radical alkyle en C₁-C₄ ou hydroxyalkyle en C₁-C₄.

L'agent oxydant présent dans la composition tinctoriale prête à l'emploi conforme à l'invention peut être choisi parmi les agents oxydants classiquement utilisés en coloration d'oxydation et de préférence parmi le peroxyde d'hydrogène, le peroxyde d'urée, les bromates de métaux alcalins, les persels tels que les perborates et persulfates. Le peroxyde d'hydrogène est particulièrement préféré.

La composition tinctoriale prête à l'emploi conforme à l'invention peut encore contenir, en plus des colorants définis ci-dessus, d'autres coupleurs et/ou des colorants directs, notamment pour modifier les nuances ou les enrichir en reflets.

Le milieu approprié pour la teinture (ou support) des compositions tinctoriales est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Les compositions tinctoriales prêtes à l'emploi conformes à l'invention peuvent également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition prête à l'emploi conforme à l'invention ne soient pas, ou substantiellement pas, altérées par la ou les adjonctions envisagées.

Les compositions tinctoriales prêtes à l'emploi conformes à l'invention peuvent se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres au moins une composition tinctoriale prête à l'emploi telle que définie précédemment, et on laisse poser pendant 3 à 50 minutes environ, de préférence 5 à 30 minutes environ, après quoi on rince, on lave éventuellement au shampooing, on rince à nouveau et on sèche.

Selon une forme de réalisation préférée, le procédé comporte une étape préliminaire consistant à stocker sous forme séparée, d'une part, une composition (A) comprenant, dans un milieu approprié pour la teinture, au moins une base d'oxydation et de la 4-hydroxyindoline et/ou au moins l'un de ses sels d'addition avec un acide et, d'autre part, une composition (B) renfermant, dans un milieu approprié pour la teinture, au moins un agent oxydant tel que par exemple défini précédemment, puis à procéder à leur mélange au moment de l'emploi avant d'appliquer ce mélange sur les fibres kératiniques, le pH des compositions (A) et (B) étant tel qu'après mélange de 10 à 90 % de la composition (A) avec 90 à 10 % de la composition (B), le pH du mélange résultant soit inférieur à 7.

La pH des compositions (A) et (B) peut être ajusté à la valeur désirée au moyen d'agents acidifiants ou éventuellement alcalinisants classiques et tels que définis précédemment.

Un autre objet de l'invention est un dispositif à plusieurs compartiments ou "kit" de teinture ou tout autre système de conditionnement à plusieurs compartiments dont un premier compartiment renferme la composition (A) telle que définie ci-dessus et un second compartiment renferme la composition oxydante (B) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES

On a préparé les compositions tinctoriales 1 (A) et 2 (A) conformes à l'invention suivantes (teneurs en grammes) :

| **COMPOSITION** | **1 (A)** | **2 (A)** |
|---|---|---|
| Paratoluylènediamine | 0,584 | |
| Dichlorhydrate de 2,6-diméthyl paraphénylènediamine | | 0,627 |
| 4-hydroxyindoline | 0,405 | 0,405 |
| Support de teinture commun (*) | (*) | (*) |
| Eau q.s.p. | 100 g | 100 g |

| | | |
|---|---|---|
| (*) support de teinture commun : - Alcool oléique polyglycérolé à 2 moles de glycérol 4,0 g - Alcool oléique polyglycérolé à 4 moles de glycérol, à 78 % de matières actives (M.A.) 5,69 g M.A. - Acide oléique 3,0 g - Amine oléique à 2 moles d'oxyde d'éthylène vendue sous la dénomination commerciale ETHOMEEN O12 par la société AKZO 7,0 g - Laurylamino succinamate de diéthylaminopropyle, sel de sodium, à 55 % de M.A. 3,0 g M.A. - Alcool oléique 5,0 g - Diéthanolamide d'acide oléique 12,0 g - Propylèneglycol 3,5 g - Alcool éthylique 7,0 g - Dipropylèneglycol 0,5 g - Monométhyléther de propylèneglycol 9,0 g - Métabisulfite de sodium en solution aqueuse, à 35 % de M.A. 0,455 g M.A. - Acétate d'ammonium 0,8 g - Antioxydant, séquestrant q.s. - Parfum, conservateur q.s. - Monoéthanolamine q.s.p. pH 9,8 | | |

Au moment de l'emploi, on a mélangé chaque composition tinctoriale 1 (A) et 2 (A) avec une quantité égale en poids d'une composition oxydante (B) constituée par une solution d'eau oxygénée à 20 volumes (6 % en poids), et dont le pH avait été ajusté entre 1 et 1,5 par 2,5 g d'acide orthophosphorique pour 100 g d'eau oxygénée.

Chaque composition résultante présentait un pH inférieur à 7 et a été appliquée pendant 30 minutes sur des mèches de cheveux gris permanentés à 90 % de blancs. Les mèches de cheveux ont ensuite été rincées, lavées avec un shampooing standard puis séchées.

Des compositions 1' (A) et 2' (A) ont également été préparées. Ces compositions 1' (A) et 2' (A) ne différaient des compositions 1 (A) et 2 (A) que par le fait que la monoéthanolamine utilisée pour ajuster le pH de la composition tinctoriale (voir le support de teinture commun défini ci-dessus) a été remplacée par 10 g d'ammoniaque à 20 % de NH₃.

Au moment de l'emploi, on a mélangé chaque composition tinctoriale 1' (A) et 2' (A) avec une quantité égale en poids d'une composition oxydante (B') constituée par une solution d'eau oxygénée à 20 volumes (6 % en poids) et de pH 3.

Chaque composition résultante (ne faisant pas partie de l'invention) présentait un pH supérieur à 7 et a été appliquée pendant 30 minutes sur des mèches de cheveux gris permanentés à 90 % de blancs. Les mèches de cheveux ont ensuite été rincées, lavées avec un shampooing standard puis séchées.

La couleur des mèches a ensuite été évaluée dans le système MUNSELL au moyen d'un colorimètre CM 2002 MINOLTA.

Selon la notation MUNSELL, une couleur est définie par l'expression H V / C dans laquelle les trois paramètres désignent respectivement la teinte ou Hue (H), l'intensité ou Value (V) et la pureté ou Chromaticité (C), la barre oblique de cette expression est simplement une convention et n'indique pas un ratio.

La différence entre la couleur des mèches avant et après la teinture a été calculée en appliquant la formule de NICKERSON :
ΔE = 0,4 C₀ΔH + 6ΔV + 3 ΔC, telle que décrite par exemple dans "Couleur, Industrie et Technique" ; pages 14-17 ; vol. n° 5 ; 1978.

Dans cette formule, ΔE représente la différence de couleur entre deux mèches, ΔH, ΔV et ΔC représentent la variation en valeur absolue des paramètres H, V et C et C₀ représente la pureté de la mèche par rapport à laquelle on désire évaluer la différence de couleur.
ΔE reflète donc la puissance de la coloration obtenue, qui est d'autant plus grande que la valeur de ΔE est élevée.

La couleur des mèches avant teinture était : 4,3 Y 4,9 / 1,4 et donc C₀ = 1,4

Les résultats sont donnés dans le tableau ci-dessous :

| **EXEMPLE [Composition]** | **pH du mélange appliqué sur les cheveux** | **Couleur de la mèche après teinture** | **Puissance de la coloration** | | | |
|---|---|---|---|---|---|---|
| | | | ΔH | ΔV | ΔC | Δ**E** |
| **1 [1 (A)]** | **6,9** | 7,8 YR 2,1/0,5 | 6,5 | 2,8 | 0,9 | **23,1** |
| 1'[1'(A)] | 9,9 | 5,2 YR 2,6/1,1 | 9,1 | 2,3 | 0,3 | 19,8 |
| **2 [2 (A)]** | **6,9** | 8,6 YR 2,3/0,3 | 5,7 | 2,6 | 1,1 | **22,1** |
| 2'[2'(A)] | 9,9 | 4,6 YR 2,7/0,8 | 9,7 | 2,2 | 0,6 | 20,4 |

Ces résultats montrent que lorsque la teinture est réalisée avec les compositions tinctoriales prêtes à l'emploi conformes à l'invention, c'est à dire dont le pH est inférieur à 7 (compositions tinctoriales prêtes à l'emploi des exemples 1 et 2), elle conduit à des colorations plus puissantes que celles obtenues avec les compositions tinctoriales prêtes à l'emploi ne faisant pas partie de l'invention (compositions tinctoriales prêtes à l'emploi des exemples 1' et 2'), qui présentent en effet un pH supérieur à 7.

## Revendications

1. Composition prête à l'emploi, pour la teinture d'oxydation des fibres kératiniques et en particulier des fibres kératiniques humaines telles que les cheveux, caractérisée par le fait qu'elle comprend, dans un milieu approprié pour la teinture :
- au moins une base d'oxydation,
- de la 4-hydroxyindoline et/ou au moins l'un des ses sels d'addition avec un acide, à titre de coupleur,
- au moins un agent oxydant,
le pH de cette composition prête à l'emploi étant inférieur à 7.

2. Composition selon la revendication 1, caractérisée par le fait que les bases d'oxydation sont choisies parmi les paraphénylènediamines, les bis-phénylalkylènediamines, les para-aminophénols, les ortho-aminophénols, les bases hétérocycliques, et leurs sels d'addition avec un acide.

3. Composition selon la revendication 2, caractérisée par le fait que les paraphénylènediamines sont choisies parmi les composés répondant à la formule (I) suivante, et leurs sels d'addition avec un acide : 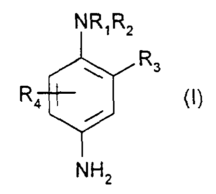 dans laquelle :
R₁ représente un atome d'hydrogène, un radical alkyle en C₁-C₄, monohydroxyalkyle en C₁-C₄, polyhydroxyalkyle en C₂-C₄, alcoxy(C₁-C₄)alkyle(C₁-C₄), phényle ou 4'-aminophényle,
R₂ représente un atome d'hydrogène, un radical alkyle en C₁-C₄, monohydroxyalkyle en C₁-C₄ ou polyhydroxyalkyle en C₂-C₄,
R₃ représente un atome d'hydrogène, un atome d'halogène tel qu'un atome de chlore, de brome, d'iode ou de fluor, un radical alkyle en C₁-C₄, monohydroxyalkyle en C₁-C₄ ou hydroxyalcoxy en C₁-C₄,
R₄ représente un atome d'hydrogène ou un radical alkyle en C₁-C₄.

4. Composition selon la revendication 3, caractérisée par le fait que les paraphénylènediamines de formule (I) sont choisies parmi la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-amino N,N-bis-(β-hydroxyéthyl) 3-méthyl aniline, la 4-amino 3-chloro N,N-bis-(β-hydroxyéthyl) aniline, la 4-amino N-(β-méthoxyéthyl) aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, et leurs sels d'addition avec un acide.

5. Composition selon la revendication 4, caractérisée par le fait que les paraphénylènediamines de formule (I) sont choisies parmi la paraphénylènediamine, la paratoluylènediamine, la 2-isopropyl paraphénylènediamine, la 2-β-hydroxyéthyl paraphénylènediamine, la 2-β-hydroxyéthyloxy paraphénylènediamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 4-amino N-(β-méthoxyéthyl) aniline, la 2-chloro paraphénylènediamine, et leurs sels d'addition avec un acide.

6. Composition selon la revendication 2, caractérisée par le fait que les bis-phénylalkylènediamines sont choisies parmi les composés de formule (II) suivante, et leurs sels d'addition avec un acide : 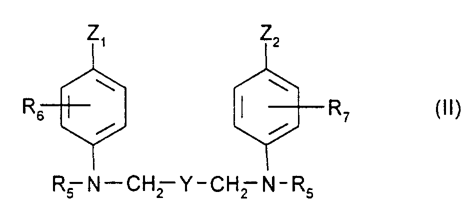 dans laquelle :
Z₁ et Z₂, identiques ou différents, représentent un radical hydroxyle ou NHR₈ dans lequel R₈ représente un atome d'hydrogène ou un radical alkyle en C₁-C₄,
R₅ représente un atome d'hydrogène, un radical alkyle en C₁-C₄, monohydroxyalkyle en C₁-C₄, polyhydroxyalkyle en C₂-C₄ ou aminoalkyle en C₁-C₄ dont le reste amino peut être substitué,
R₆ et R₇, identiques ou différents, représentent un atome d'hydrogène ou d'halogène ou un radical alkyle en C₁-C₄,
Y représente un radical pris dans le groupe constitué par les radicaux suivants : 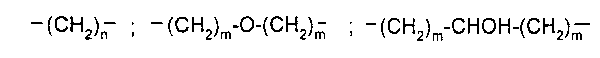 et 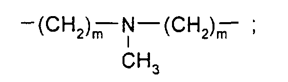 dans lesquels n est un nombre entier compris entre 0 et 8 inclusivement et m est un nombre entier compris entre 0 et 4 inclusivement.

7. Composition selon la revendication 6, caractérisée par le fait que les bis-phénylalkylènediamines sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

8. Composition selon la revendication 7, caractérisée par le fait que les bis-phénylalkylènediamines sont choisies parmi le N,N'-bis-(β-hydroxyéthyl) N,N'-bis-(4'-aminophényl) 1,3-diamino propanol, et ses sels d'addition avec un acide.

9. Composition selon la revendication 2, caractérisée par le fait que les para-aminophénols sont choisis parmi les composés répondant à la formule (III) suivante, et leurs sels d'addition avec un acide : 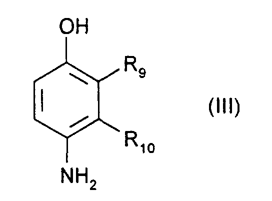 dans laquelle :
R₉ représente un atome d'hydrogène, un radical alkyle en C₁-C₄, monohydroxyalkyle en C₁-C₄, alcoxy(C₁-C₄)alkyle(C₁-C₄) ou aminoalkyle en C₁-C₄,
R₁₀ représente un atome d'hydrogène ou de fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C4)alkyle(C₁-C₄),
étant entendu qu'au moins un des radicaux R₉ ou R₁₀ représente un atome d'hydrogène.

10. Composition selon la revendication 9, caractérisée par le fait que les para-aminophénols sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.

11. Composition selon la revendication 2, caractérisée par le fait que les orthoaminophénols sont choisis parmi le 2-amino phénol, le 2-amino 5-méthyl phénol, le 2-amino 6-méthyl phénol, le 5-acétamido 2-amino phénol, et leurs sels d'addition avec un acide.

12. Composition selon la revendication 2 , caractérisée par le fait que les bases hétérocycliques sont choisies parmi les dérivés pyridiniques, les dérivés pyrimidiniques, les dérivés pyrazoliques, et leurs sels d'addition avec un acide.

13. Composition selon la revendication 12, caractérisée par le fait que les bases d'oxydation hétérocycliques sont choisies parmi la 2,4,5,6-tétraaminopyrimidine, la 2,5-diaminopyridine, le 4,5-diamino 1-méthyl pyrazole, le 3,4-diamino pyrazole, le 4,5-diamino 1-(4'-chlorobenzyl) pyrazole, la 4-hydroxy 2,5,6-triaminopyrimidine, et leurs sels d'addition avec un acide.

14. Composition selon l'une quelconque des revendications précédentes, caractérisée par le fait que les sels d'addition avec un acide sont choisis parmi les chlorhydrates, les bromhydrates, les sulfates et les tartrates.

15. Composition selon l'une quelconque des revendications précédentes, caractérisée par le fait que la ou les bases d'oxydation représentent de 0,0005 à 12 % en poids du poids total de la composition tinctoriale.

16. Composition selon la revendication 15, caractérisée par le fait que la ou les bases d'oxydation représentent de 0,005 à 6 % en poids du poids total de la composition tinctoriale.

17. Composition selon l'une quelconque des revendications précédentes, caractérisée par le fait que la 4-hydroxyindoline et/ou ses sels d'addition avec un acide représente(nt) de 0,0005 à 5 % en poids du poids total de la composition tinctoriale.

18. Composition selon la revendication 17, caractérisée par le fait que la 4-hydroxyindoline et/ou ses sels d'addition avec un acide représente(nt) de 0,005 à 3 % en poids du poids total de la composition tinctoriale.

19. Composition selon l'une quelconque des revendications précédentes, caractérisée par le fait qu'elle présente un pH compris entre 3 et 6,9.

20. Composition selon l'une quelconque des revendications précédentes, caractérisée par le fait que l'agent oxydant est choisi parmi le peroxyde d'hydrogène, le peroxyde d'urée, les bromates de métaux alcalins, les persels tels que les perborates et persulfates.

21. Procédé de teinture des fibres kératiniques et en particulier des fibres kératiniques humaines telles que les cheveux, caractérisé par le fait que l'on applique sur lesdites fibres au moins composition tinctoriale prête à l'emploi telle que définie à l'une quelconque des revendications précédentes.

22. Procédé selon la revendication 21, caractérisé par le fait qu'il comporte une étape préliminaire consistant à stocker sous forme séparée, d'une part, une composition (A) comprenant, dans un milieu approprié pour la teinture, au moins une base d'oxydation et de la 4-hydroxyindoline et/ou au moins l'un de ses sels d'addition avec un acide et, d'autre part, une composition (B) renfermant, dans un milieu approprié pour la teinture, au moins un agent oxydant puis à procéder à leur mélange au moment de l'emploi avant d'appliquer ce mélange sur les fibres kératiniques, le pH des compositions (A) et (B) étant tel qu'après mélange de 10 à 90 % de la composition (A) avec 90 à 10 % de la composition (B), le pH du mélange résultant soit inférieur à 7.

23. Dispositif à plusieurs compartiments, ou "kit" de teinture, caractérisé par le fait qu'il comporte un premier compartiment renfermant la composition (A) telle que définie dans la revendication 22 et un second compartiment renfermant la composition oxydante (B) telle que définie dans la revendication 22.

## Patentansprüche

1. Gebrauchsfertige Zusammensetzung zum oxidativen Färben von Keratinfasern und insbesondere menschlichen Keratinfasern, wie dem Haar, dadurch gekennzeichnet, daß sie in einem zum Färben geeigneten Medium enthält:
- mindestens eine Oxidationsbase,
- 4-Hydroxyindolin und/oder mindestens ein Additionssalz dieser Verbindung mit einer Säure als Kuppler, und
- mindestens ein Oxidationsmittel,
- wobei der pH-Wert der gebrauchsfertigen Zusammensetzung unter 7 liegt.

2. Zusammensetzung nach Anspruch 1, dadurch gekennzeichnet, daß die Oxidationsbasen unter den p-Phenylendiaminen, Bisphenylalkylendiaminen, p-Aminophenolen, o-Aminophenolen, heterocyclischen Basen und deren Additionssalzen mit einer Säure ausgewählt sind.

3. Zusammensetzung nach Anspruch 2, dadurch gekennzeichnet, daß die p-Phenylendiamine unter den Verbindungen der folgenden Formel (I) und deren Additionssalzen mit einer Säure ausgewählt sind: 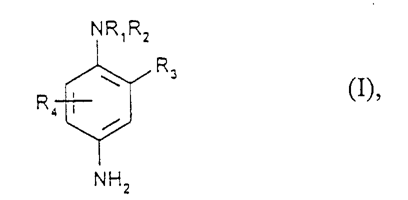 worin bedeuten:
R₁ Wasserstoff, C₁₋₄-Alkyl, C₁₋₄-Monohydroxyalkyl, C₂₋₄-Polyhydroxyalkyl, C₁₋₄-Alkoxy-C₁₋₄-alkyl, Phenyl oder 4'-Aminophenyl,
R₂ Wasserstoff, C₁₋₄-Alkyl, C₁₋₄-Monohydroxyalkyl oder C₂₋₄-Polyhydroxyalkyl,
R₃ Wasserstoff, Halogen, wie Chlor, Brom, Iod oder Fluor, C₁₋₄-Alkyl, C₁₋₄-Monohydroxyalkyl oder C₁₋₄-Hydroxyalkoxy, und
R₄ Wasserstoff oder C₁₋₄-Alkyl.

4. Zusammensetzung nach Anspruch 3, dadurch gekennzeichnet, daß die p-Phenylendiamine der Formel (I) unter p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-Amino-N,N-bis(β-hydroxyethyl)-3-methyl-anilin, 4-Amino-3-chlor-N,N-bis(β-hydroxyethyl)-anilin, 4-Amino-N-(ß-methoxyethyl)-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-β-Hydroxyethyloxy-p-phenylendiamin und deren Additionssalzen mit einer Säure ausgewählt sind.

5. Zusammensetzung nach Anspruch 4, dadurch gekennzeichnet, daß die p-Phenylendiamine der Formel (I) unter p-Phenylendiamin, p-Toluylendiamin, 2-Isopropyl-p-phenylendiamin, 2-β-Hydroxyethyl-p-phenylendiamin, 2-β-Hydroxyethyloxy-p-phenylendiamin, 2,6-Dimethyl-p-phenylendiamin, 2,6-Diethyl-p-phenylendiamin, 2,3-Dimethyl-p-phenylendiamin, N,N-Bis(β-hydroxyethyl)-p-phenylendiamin, 4-Amino-N-(β-methoxyethyl)-anilin, 2-Chlor-p-phenylendiamin und deren Additionssalzen mit einer Säure ausgewählt sind.

6. Zusammensetzung nach Anspruch 2, dadurch gekennzeichnet, daß die Bisphenylalkylendiamine unter den Verbindungen der folgenden Formel (II) und deren Additionssalze mit einer Säure ausgewählt sind: 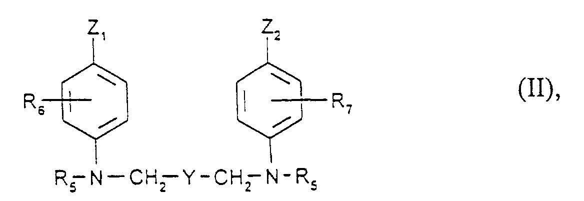 worin bedeuten:
Z₁ und Z₂, die identisch oder voneinander verschieden sind, Hydroxy oder NHR₈, wobei R₈ Wasserstoff oder C₁₋₄-Alkyl bedeutet,
R₅ Wasserstoff, C₁₋₄-Alkyl, C₁₋₄-Monohydroxyalkyl, C₂₋₄-Polyhydroxyalkyl oder C₁₋₄-Aminoalkyl, wobei die Aminogruppe substituiert sein kann,
R₆ und R₇, die identisch oder voneinander verschieden sind, Wasserstoff, Halogen oder C₁₋₄-Alkyl,
Y eine Gruppe, die unter den folgenden Gruppen ausgewählt ist:
-(CH₂)ₙ-; -(CH₂)ₘ-O-(CH₂)ₘ-; -(CH₂)ₘ-CHOH-(CH₂)ₘ- und 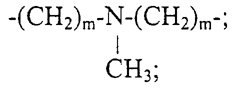
worin n Null oder eine ganze Zahl von 1 bis 8 und m Null oder eine ganze Zahl im Bereich von 1 bis 4 bedeutet.

7. Zusammensetzung nach Anspruch 6, dadurch gekennzeichnet, daß die Bisphenylalkylendiamine unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis-(4'-amino, 3'-methylphenyl)-ethylendiamin und deren Additionssalzen mit einer Säure ausgewählt sind.

8. Zusammensetzung nach Anspruch 7, dadurch gekennzeichnet, daß die Bisphenylalkylendiamine unter N,N'-Bis(β-hydroxyethyl)-N,N'-bis(4'-aminophenyl)-1,3-diaminopropanol oder den Additionssalzen dieser Verbindung mit einer Säure ausgewählt sind.

9. Zusammensetzung nach Anspruch 2, dadurch gekennzeichnet, daß die p-Aminophenole unter den Verbindungen der folgenden Formel (III) und deren Additionssalze mit einer Säure ausgewählt sind: 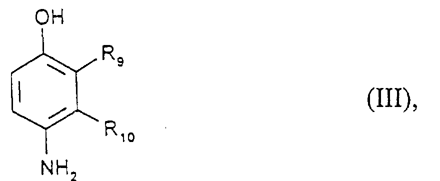 worin bedeuten:
R₉ Wasserstoff, C₁₋₄-Alkyl, C₁₋₄-Monohydroxyalkyl, C₁₋₄-Alkoxy-C₁₋₄-alkyl oder C₁₋₄-Aminoalkyl,
R₁₀ Wasserstoff, Fluor, C₁₋₄-Alkyl, C₁₋₄-Monohydroxyalkyl, C₂₋₄-Polyhydroxyalkyl, C₁₋₄-Aminoalkyl, C₁₋₄-Cyanoalkyl oder C₁₋₄-Alkoxy-C₁₋₄-alkyl,
mit der Maßgabe, daß mindestens eine der Gruppen R₉ oder R₁₀ Wasserstoff bedeutet.

10. Zusammensetzung nach Anspruch 9, dadurch gekennzeichnet, daß die p-Aminophenole unter p-Aminophenol, 4-Amino-3-methyl-phenol, 4-Amino-3-fluorphenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol und deren Additionssalzen mit einer Säure ausgewählt sind.

11. Zusammensetzung nach Anspruch 2, dadurch gekennzeichnet, daß die o-Aminophenole unter 2-Aminophenol, 2-Amino-5-methyl-phenol, 2-Amino-6-methyl-phenol, 5-Acetamido-2-amino-phenol und deren Additionssalzen mit einer Säure ausgewählt sind.

12. Zusammensetzung nach Anspruch 2, dadurch gekennzeichnet, daß die heterocyclischen Basen unter den Pyridinderivaten, Pyrimidinderivaten, die Pyrazolderivaten und deren Additionssalzen mit einer Säure ausgewählt sind.

13. Zusammensetzung nach Anspruch 12, dadurch gekennzeichnet, daß die heterocyclischen Basen unter 2,4,5,6-Tetraaminopyrimidin, 2,5-Diaminopyridin, 4,5-Diamino-1-methylpyrazol, 3,4-Diaminopyrazol, 4,5-Diamino-1-(4'-chlorbenzyl)pyrazol, 4-Hydroxy-2,5,6-triaminopyrimidin und deren Additionssalzen mit einer Säure ausgewählt sind.

14. Zusammensetzung nach einem der vorhergehenden Ansprüche, dadurch gekennzeichnet, daß die Additionssalze mit einer Säure unter den Hydrochloriden, Hydrobromiden, Sulfaten und Tartraten ausgewählt sind.

15. Zusammensetzung nach einem der vorhergehenden Ansprüche, dadurch gekennzeichnet, daß die Oxidationsbase(n) 0,0005 bis 12 Gew.-% des Gesamtgewichts der Färbemittelzusammensetzung ausmachen.

16. Zusammensetzung nach Anspruch 15, dadurch gekennzeichnet, daß die Oxidationsbase(n) 0,005 bis 6 Gew.-% des Gesamtgewichts der Färbemittelzusammensetzung ausmachen.

17. Zusammensetzung nach einem der vorhergehenden Ansprüche, dadurch gekennzeichnet, daß das 4-Hydroxyindolin und/oder seine Additionssalze mit einer Säure 0,0005 bis 5 Gew.-% des Gesamtgewichts der Färbemittelzusammensetzung ausmacht (ausmachen).

18. Zusammensetzung nach Anspruch 17, dadurch gekennzeichnet, daß das 4-Hydroxyindolin und/oder seine Additionssalze mit einer Säure 0,005 bis 3 Gew.-% des Gesamtgewichts der Färbemittelzusammensetzung ausmacht (ausmachen).

19. Zusammensetzung nach einem der vorhergehenden Ansprüche, dadurch gekennzeichnet, daß sie einen pH-Wert von 3 bis 6,9 aufweist.

20. Zusammensetzung nach einem der vorhergehenden Ansprüche, dadurch gekennzeichnet, daß das Oxidationsmittel unter Wasserstoffperoxid, Harnstoffperoxid, Alkalimetallbromaten und Salzen von Persäuren, wie Perboraten und Persulfaten, ausgewählt ist.

21. Verfahren zum Färben von Keratinfasern und insbesondere menschlichen Keratinfasern, wie dem Haar, dadurch gekennzeichnet, daß auf die Fasern mindestens eine gebrauchsfertige Färbemittelzusammensetzung nach einem der vorhergehenden Ansprüche aufgebracht wird.

22. Verfahren nach Anspruch 21, dadurch gekennzeichnet, daß es einen vorbereiteten Schritt umfaßt, der darin besteht, einerseits eine Zusammensetzung (A), die in einem zum Färben geeigneten Medium mindestens eine Oxidationsbase und 4-Hydroxyindolin und/oder mindestens eines seiner Additionssalze mit einer Säure enthält, und andererseits eine Zusammensetzung (B) getrennt voneinander aufzubewahren, die in einem zum Färben geeigneten Medium mindestens ein Oxidationsmittel enthält, und diese bei der Anwendung zu vermischen, bevor das Gemisch auf die Keratinfasern aufgebracht wird, wobei der pH-Wert der beiden Zusammensetzungen (A) und (B) so ist, daß nach dem Mischen von 10 bis 90 % Zusammensetzung (A) mit 90 bis 10 % Zusammensetzung (B) der pH-Wert des resultierenden Gemisches unter 7 liegt.

23. Vorrichtung mit mehreren Abteilungen oder "Kit" zum Färben, dadurch gekennzeichnet, daß sie eine erste Abteilung mit der in Anspruch 22 definierten Zusammensetzung (A) und eine zweite Abteilung mit der in Anspruch 22 definierten oxidierenden Zusammensetzung (B) aufweist.

## Claims

1. Ready-to-use composition for the oxidation dyeing of keratin fibres, and in particular of human keratin fibres such as the hair, characterized in that it comprises, in a medium which is suitable for dyeing:
- at least one oxidation base,
- 4-hydroxyindoline and/or at least one of the addition salts thereof with an acid, as coupler,
- at least one oxidizing agent,
the pH of this ready-to-use composition being less than 7.

2. Composition according to Claim 1, characterized in that the oxidation bases are chosen from para-phenylenediamines, bis(phenyl)alkylenediamines, para-aminophenols, ortho-aminophenols and heterocyclic bases, and the addition salts thereof with an acid.

3. Composition according to Claim 2, characterized in that the para-phenylenediamines are chosen from compounds corresponding to formula (I) below, and the addition salts thereof with an acid: 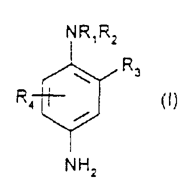 in which:
R₁ represents a hydrogen atom or a C₁-C₄ alkyl, C₁-C₄ monohydroxyalkyl, C₂-C₄ polyhydroxyalkyl, (C₁-C₄) alkoxy-(C₁-C₄) alkyl, phenyl or 4'-aminophenyl radical,
R₂ represents a hydrogen atom or a C₁-C₄ alkyl, C₁-C₄ monohydroxyalkyl or C₂-C₄ polyhydroxyalkyl radical,
R₃ represents a hydrogen atom, a halogen atom such as a chlorine, bromine, iodine or fluorine atom, or a C₁-C₄ alkyl, C₁-C₄ monohydroxyalkyl or C₁-C₄ hydroxyalkoxy radical,
R₄ represents a hydrogen atom or a C₁-C₄ alkyl radical.

4. Composition according to Claim 3, characterized in that the para-phenylenediamines of formula (I) are chosen from para-phenylenediamine, para-tolylenediamine, 2-chloro-para-phenylenediamine, 2,3-dimethylpara-phenylenediamine, 2,6-dimethyl-para-phenylenediamine, 2,6-diethyl-para-phenylenediamine, 2,5-dimethylpara-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis (β-hydroxyethyl)-para-phenylenediamine, 4-amino-N,N-bis (β-hydroxyethyl) -3-methyl-aniline, 4-amino-3-chloro-N,N-bis (β-hydroxyethyl)aniline, 4-amino-N- (β-methoxyethyl) aniline, 2-β-hydroxyethyl-para-phenylenediamine, 2-fluoro-paraphenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine, N-ethyl-N-(β-hydroxyethyl)-para-phenylenediamine, N-(β,γ-dihydroxypropyl)-paraphenylenediamine, N-(4'-aminophenyl)-para-phenylenediamine, N-phenyl-para-phenylenediamine and 2-β-hydroxyethyloxy-para-phenylenediamine, and the addition salts thereof with an acid.

5. Composition according to Claim 4, characterized in that the para-phenylenediamines of formula (I) are chosen from para-phenylenediamine, para-tolylenediamine, 2-isopropyl-para-phenylenediamine, 2-β-hydroxyethyl-para-phenylenediamine, 2-β-hydroxyethyloxy-para-phenylenediamine, 2,6-dimethyl-para-phenylenediamine, 2,6-diethyl-para-phenylenediamine, 2,3-dimethyl-para-phenylenediamine, N,N-bis (β-hydroxyethyl)-para-phenylenediamine, 4-amino-N- (β-methoxyethyl)aniline and 2-chloro-para-phenylenediamine, and the addition salts thereof with an acid.

6. Composition according to Claim 2, characterized in that the bis(phenyl)alkylenediamines are chosen from the compounds of formula (II) below, and the addition salts thereof with an acid: 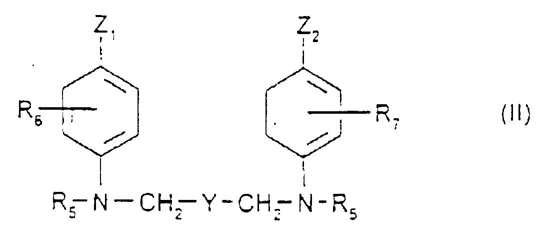 in which:
Z₁ and Z₂, which may be identical or different, represent a hydroxyl radical or a radical NHR₈ in which R₈ represents a hydrogen atom or a C₁-C₄ alkyl radical,
R₅ represents a hydrogen atom or a C₁-C₄ alkyl, C₁-C₄ monohydroxyalkyl or C₂-C₄ polyhydroxyalkyl radical or a
C₁-C₄ aminoalkyl radical in which the amino residue may be substituted,
R₆ and R₇, which may be identical or different, represent a hydrogen or halogen atom or a C₁-C₄ alkyl radical,
Y represents a radical taken from the group consisting of the following radicals:
- (CH₂)ₙ- ; - (CH₂)ₘ-O- (CH₂)ₘ- ; - (CH₂)ₘ-CHOH- (CH₂)ₘ- and 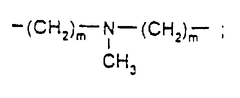 in which n is an integer between 0 and 8 inclusive and m is an integer between 0 and 4 inclusive.

7. Composition according to Claim 6, characterized in that the bis(phenyl)alkylenediamines are chosen from N,N'-bis(β-hydroxyethyl)-N,N'-bis(4'-aminophenyl)-1,3-diaminopropanol, N,N'-bis (β-hydroxyethyl)-N,N'-bis (4'-aminophenyl)ethylenediamine, N,N'-bis(4-aminophenyl)tetramethylenediamine, N,N'-bis (β-hydroxyethyl)-N,N'-bis(4-aminophenyl)tetramethylenediamine, N,N'-bis(4-methylaminophenyl)tetramethylenediamine and N,N'-bis(ethyl)-N,N'-bis(4'-amino-3'-methylphenyl)ethylenediamine, and the addition salts thereof with an acid.

8. Composition according to Claim 7, characterized in that the bis(phenyl)alkylenediamines are chosen from N,N'-bis(β-hydroxyethyl)-N,N'-bis(4'-aminophenyl)-1,3-diaminopropanol, and the addition salts thereof with an acid.

9. Composition according to Claim 2, characterized in that the para-aminophenols are chosen from the compounds corresponding to formula (III) below, and the addition salts thereof with an acid: 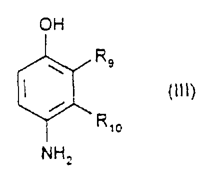 in which:
R₉ represents a hydrogen atom or a C₁-C₄ alkyl, C₁-C₄ monohydroxyalkyl, (C₁-C₄) alkoxy(C₁-C₄) alkyl or C₁-C₄ aminoalkyl radical,
R₁₀ represents a hydrogen or fluorine atom or a C₁-C₄ alkyl, C₁-C₄ monohydroxyalkyl, C₂-C₄ polyhydroxyalkyl,
C₁-C₄ aminoalkyl, cyano (C₁-C₄) alkyl or (C₁-C₄)alkoxy-(C₁-C₄) alkyl radical,
it being understood that at least one of the radicals R₉ or R₁₀ represents a hydrogen atom.

10. Composition according to Claim 9, characterized in that the para-aminophenols are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol and 4-amino-2-(β-hydroxyethylaminomethyl) phenol, and the addition salts thereof with an acid.

11. Composition according to Claim 2, characterized in that the ortho-aminophenols are chosen from 2-aminophenol, 2-amino-5-methylphenol, 2-amino-6-methylphenol and 5-acetamido-2-aminophenol, and the addition salts thereof with an acid.

12. Composition according to Claim 2, characterized in that the heterocyclic bases are chosen from pyridine derivatives, pyrimidine derivatives and pyrazole derivatives, and the addition salts thereof with an acid.

13. Composition according to Claim 12, characterized in that the heterocyclic oxidation bases are chosen from 2,4,5,6-tetraaminopyrimidine, 2,5-diaminopyridine, 4,5-diamino-1-methylpyrazole, 3,4-diaminopyrazole, 4,5-diamino-1-(4'-chlorobenzyl)pyrazole and 4-hydroxy-2,5,6-triaminopyrimidine, and the addition salts thereof with an acid.

14. Composition according to any one of the preceding claims, characterized in that the addition salts with an acid are chosen from the hydrochlorides, hydrobromides, sulphates and tartrates.

15. Composition according to any one of the preceding claims, characterized in that the oxidation base (s) represent (s) from 0.0005% to 12% by weight relative to the total weight of the dye composition.

16. Composition according to Claim 15, characterized in that the oxidation base(s) represent(s) from 0.005% to 6% by weight relative to the total weight of the dye composition.

17. Composition according to any one of the preceding claims, characterized in that the 4-hydroxyindoline and/or the addition salts thereof with an acid represent (s) from 0.0005% to 5% by weight relative to the total weight of the dye composition.

18. Composition according to Claim 17, characterized in that the 4-hydroxyindoline and/or the addition salts thereof with an acid represent(s) from 0.005% to 3% by weight relative to the total weight of the dye composition.

19. Composition according to any one of the preceding claims, characterized in that it has a pH of between 3 and 6.9.

20. Composition according to any one of the preceding claims, characterized in that the oxidizing agent is chosen from hydrogen peroxide, urea peroxide, alkali metal bromates and persalts such as perborates and persulphates.

21. Process for dyeing keratin fibres, and in particular human keratin fibres such as the hair, characterized in that at least one ready-to-use dye composition as defined in any one of the preceding claims is applied to the said fibres.

22. Process according to Claim 21, characterized in that it comprises a preliminary step which consists in separately storing, on the one hand, a composition (A) comprising, in a medium which is suitable for dyeing, at least one oxidation base and 4-hydroxyindoline and/or at least one of the addition salts thereof with an acid and, on the other hand, a composition (B) containing, in a medium which is suitable for dyeing, at least one oxidizing agent, and then in mixing them together at the time of use, followed by applying this mixture to the keratin fibres, the pH of compositions (A) and (B) being such that after mixing from 10% to 90% of composition (A) with 90% to 10% of composition (B), the pH of the resulting mixture is less than 7.

23. Multi-compartment device, or dyeing "kit", characterized in that it comprises a first compartment containing the composition (A) as defined in Claim 22 and a second compartment containing the oxidizing composition (B) as defined in Claim 22.
